Europäisches Patentamt

European Patent Office

Office européen des brevets

(11) Publication number: **0 227 219**
**B1**

(12) **EUROPEAN PATENT SPECIFICATION**

(45) Date of publication of patent specification: **19.12.90**

(21) Application number: **86307462.1**

(22) Date of filing: **29.09.86**

(51) Int. Cl.⁵: **A 61 M 39/00,** A 61 M 1/28, A 61 M 5/00

(54) Sterile connection apparatus.

(30) Priority: **30.09.85 JP 218714/85**

(43) Date of publication of application: **01.07.87 Bulletin 87/27**

(45) Publication of the grant of the patent: **19.12.90 Bulletin 90/51**

(84) Designated Contracting States: **DE FR GB SE**

(56) References cited:
DE-A-3 043 333
FR-A-2 455 462
FR-A-2 486 803
FR-A-2 529 788
GB-A-2 060 399
US-A-4 209 013
US-A-4 403 992

(73) Proprietor: **Japan Medical Supply Company Limited**
**12-17 Kako-Machi Naka-ku**
**Hiroshima City Hiroshima Pref. (JP)**

(72) Inventor: **Kato ,Yasuaki**
**1-13-12 Omiya-cho**
**Nishi-ku Hiroshima, 733 (JP)**

(74) Representative: **Crisp, David Norman et al**
**D. YOUNG & CO. 10 Staple Inn**
**London, WC1V 7RD (GB)**

Courier Press, Leamington Spa, England.

## Description

This invention relates to sterile connection apparatus for connecting medical tubing via a connector maintained in a sterile condition.

In many medical situations, it is often necessary to connect tubes under sterile conditions. For example, in peritoneal dialysis for removing unwanted substances from blood through the peritoneum using a dialysate injected into the peritoneal cavity, the connection of tubes under sterile conditions is extremely important. The dialysate must be injected into the peritoneal cavity and drained therefrom under sterile conditions because the peritoneal cavity is easily infected with pathogenic bacteria which cause peritonitis. In CAPD (Continuous Ambulatory Peritoneal Dialysis) which has become widely used for peritoneal dialysis, the dialysate replaced four times a day but there is still the possibility of bacterial infection at the time of changing the connection between the peritoneal catheter and the dialysate bag and, therefore, a sterile connection process is especially required. Furthermore, in intravenous hyperalimentation, a connector is used to connect a catheter and a bag and it is necessary to effect sterile connection in order to prevent bacterial infection at the time of changing the connection.

In previous sterile connection processes it has been proposed to sterilise the connector with an antiseptic solution, by heating, or by irradiation with ultraviolet rays. In particular, disinfection of the connector with an antiseptic solution is widely used since it requires no complicated apparatus and is simple to operate. However, bacterial infection is still readily possible. Accordingly, various disinfection apparatus for reducing infection have been proposed. For example, DE-A-3,043,333, FR-A-2,4555,462, US 4,209,013 and GB-A-2,060,399, disclose apparatus for effecting connection using a connector hermetically sealed in a container filled with an antiseptic solution. However, these apparatus are difficult to work with and have high manufacturing costs.

As a result of various investigations into the causes of bacterial infection when conventional disinfection apparatus are used, we have now found that imperfect disinfection of the connector and the recontamination thereof after disinfection caused bacterial infection. In particular, it has been found that recontamination of the connector occurs when the connector is accidentally contacted with a non-sterile part of the apparatus at the time of detachment of the connector from the disinfector.

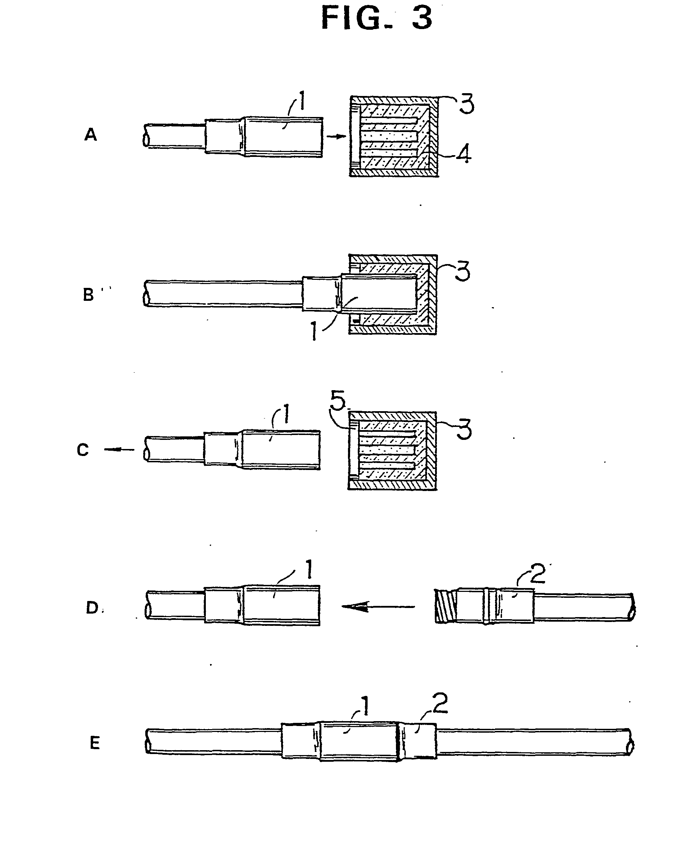

A previously proposed disinfection process is illustrated with reference to Figure 3 of the accompanying drawings. A first connector member 1 connected to a catheter or the extension tube thereof is repeatedly used and, because of this, the outer peripheral part thereof may well be highly contaminated with bacteria. Therefore, said first connector member 1 should be disinfected before being coupled with a second connector member 2.

In disinfection, the member 1 is first inserted into a disinfection container 3 having a liquid absorbant material 4 impregnated with an antiseptic solution (Figure A). After the member 1 is allowed to stand for an appropriate time (Figure B), the member 1 is pulled from the container 3 (Figure C) and then connected to the connector member 2 (Figure D, E). However, the end part of the member 1 contacts the non-disinfected part 5 of the container 3 when the member 1 is pulled from the container 3. The non-disinfected part 5 may be subject to bacterial contamination because there is no adhesion of antiseptic solution and there is therefore the possibility of recontamination if the connector member 1 contacts said part 5.

FR-A-2,529,788 describes apparatus to protect a connection between tubular conduits comprising a shell having at least a first opening allowing the connection to pass between the interior and exterior of the shell, at least a second opening allowing one of the tubular conduits to be joined to pass therethrough, and an elastic substrate within the shell capable of releasing an antiseptic material.

It has also been proposed to spray an antiseptic solution onto the connector and to wind a sponge or the like impregnated with an antiseptic solution around the outer peripheral part of the connector, but proper sterilization cannot be achieved by these methods.

We have now found it possible to provide apparatus to enable sterile connection of a connector without the possibility of recontamination. The apparatus is simple to operate and enables proper disinfection.

In accordance with the invention, there is provided a sterile connection apparatus for the sterile connection of a first connector to a second connector, comprising a side wall member, a first opening in the side wall member opposite to a second opening, and liquid absorbent materials impregnated with an antiseptic solution, wherein the first opening is of a size sufficient for the insertion of the first connector, the inserted first connector thereupon being contacted with the liquid absorbent materials, and the second opening having a size sufficient for the insertion of the second connector, characterised in that a bottom member is detachably attached to the side wall member whereby the second opening is cleared when the said bottom member is detached from the side wall member and whereby a part of the absorbent materials is fixed to the bottom member to disinfect the end part and the interior of the first connector.

In connecting a first connector which may be contaminated at its end to a second connector under sterile conditions using the apparatus of the present invention, the first connector is inserted into the opening so that its end is contacted with the liquid absorbant materials and the bottom member is subsequently detached to form a second opening for insertion of the second connector and thus complete the connection of the two connectors.

Embodiments of the invention are hereinafter described by way of non-limiting example with reference to the accompanying drawings. In the description below, CAPO is referred to but the invention is not limited thereto. In the drawings:-

Figure 1 is a partial cross-sectional view of a connection process using a sterile connection apparatus of this invention;

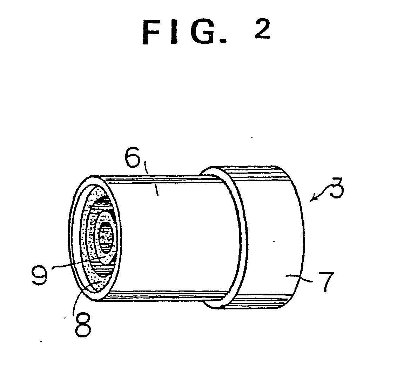

Figure 2 is a perspective view of a sterile connection apparatus of this invention; and

Figure 3 is a partial cross sectional view of a connection process using a previously proposed apparatus as described above.

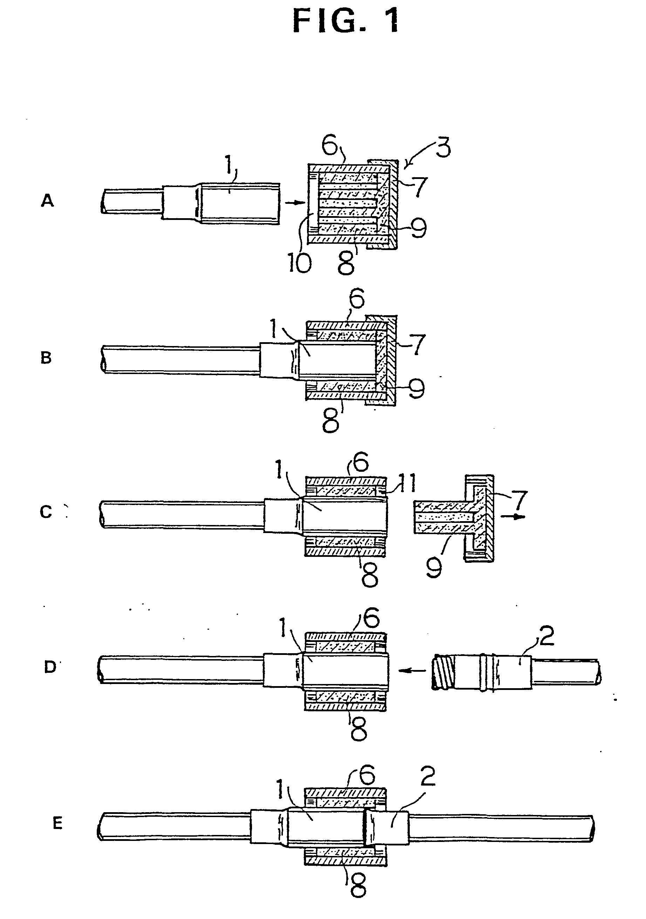

With reference to Figure 1, a first connector 1 is connected, for example, to a peritoneal catheter (not shown) or an extension tube therefor and may be used repeatedly. During a dialysis operation, the connector 1 is connected to a second connector 2 which is itself connected to a dialysate bag (also not shown). In replacing the dialysate bag with a new one, the first connector is first separated from the second connector and it may be disinfected using the apparatus 3 of the invention. The apparatus 3 has a side wall member 6, a bottom member 7 and an opening 10 opposite to the bottom member 7. The bottom member 7 is detachably attached to the side wall member 6, and liquid absorbant materials 8, 9 impregnated with an antiseptic solution are secured to the apparatus 3. The side wall member 6 fits into the bottom member 7. The liquid absorbant material 8 has a cylindrical shape and is fixed to the side wall member 6, while the liquid absorbant material 9 is fixed to the bottom member 7 and has the shape of a cylinder bonded to a disc.

In use, the first connector 1 is inserted into the opening 10 of the apparatus 3 (Figure 1A) and contacted with the liquid absorbant materials 8, 9 for a sufficient time to effect disinfection (Figure 1B). In this way the outer periphery of the connector 1 is disinfected by the liquid absorbant material 8 and the end part and interior thereof are disinfected by the liquid absorbant material 9. Next, the connector 1 is pushed further into the apparatus 3 to detach the bottom member 7 and a second opening 11 is uncovered (Figure 1C). Then, the second connector 2 connected to a new dialysate bag is connected to the first connector member 1 (Figures 1D and 1E). The connector member 2 is pre-sterilized and it is therefore unnecessary to disinfect this prior to connection. After the connection process is completed, the connected part is surrounded by the side wall member 6 and the liquid absorbant material 8 which protects against further contamination.

As will be appreciated by the foregoing explanation, because the disinfected connector members are not removed from apparatus in the invention, there is no possibility of recontamination. Further, the disinfecting and connecting operations are extremely simple.

The apparatus 3 is conveniently selected so that the size thereof coincides with that of the connector to be disinfected. That is, the inner diameter of the liquid absorbant material 8 is approximately equal to the outer diameter of the connector. The liquid absorbant material 9 is preferably selected so that the shape thereof coincides with the internal shape of the first connector 1. Examples of liquid absorbant materials which may be used include synthetic resin foams or nonwoven fabrics, and preferably synthetic resin foams which may be readily moulded. Flexible polyurethane foam is most preferred.

In use of the apparatus of the invention, the connectors are connected together and may be used as shown in Figure 1E. If desired, a cover may be further attached thereon to protect the side wall member 6. By attaching such a cover, the side wall member 6 can be fixed in place.

The antiseptic solution for use in accordance with the invention may be conveniently selected from solution of Pobidon iodine, chlorohexidine gluconate, benzalconium chloride, isopropanol and mixtures thereof.

In accordance with the invention, not only can disinfection of the connector be properly performed in a simple operation, but also the disinfected connector is not likely to be recontaminated. In addition, because the connector is surrounded over its circumference by the apparatus after connection and protected thereby, the connection can be kept clean until the next connection is performed. Therefore, the sterile connection apparatus of the invention can be preferably used in peritoneal dialysis or intravenous hyperalimentation.

**Claims**

1. A sterile connection apparatus for the sterile connection of a first connector (1) to a second connector (2), comprising a side wall member (6), a first opening (10) in the side wall member (6) opposite to a second opening (11), and liquid absorbent materials (8, 9) impregnated with an antiseptic solution, wherein the first opening (10) is of a size sufficient for the insertion of the first connector (1), the inserted first connector (1) thereupon being contacted with the liquid absorbent materials (8, 9), and the second opening (11) having a size sufficient for the insertion of the second connector (2), characterised in that a bottom member (7) is detachably attached to the side wall member (6) whereby the second opening (11) is cleared when the said bottom member (7) is detached from the side wall member (6) and whereby a part (9) of the absorbent materials (8, 9) is fixed to the bottom member (7) to disinfect the end part and the interior of the first connector (1).

2. A sterile connection apparatus as claimed in claim 1, wherein the liquid absorbant materials (8, 9) have a shape coinciding with that of the connector (1).

3. A sterile connection apparatus as claimed in either of claims 1 and 2 wherein the liquid absorbant materials (8, 9) comprise a part (8) fixed to the side wall member (6) and a part (9) fixed to the bottom member (7).

4. A sterile connection apparatus as claimed in any one of the preceding claims wherein the liquid absorbant materials (8, 9) comprise polyurethane foam.

5. A sterile connection apparatus as claimed in any one of the preceding claims, wherein the shape of the side wall member (6) is cylindrical.

6. A sterile connection apparatus as claimed in any one of the preceding claims, wherein the side wall member (6) fits into the bottom member (7).

**Patentansprüche**

1. Sterile Verbindungsvorrichtung zur sterilen Verbindung eines ersten Verbindungsstückes (1) mit einem zweiten Verbindungsstück (2) mit einem Seitenwandteil (6), einer ersten Öffnung (10) in dem Seitenwandteil (6) gegenüber einer zweiten Öffnung (11) und flüssigkeitsabsorbierenden Materialien (8, 9), die mit einer antiseptischen Lösung imprägniert sind, wobei die erste Öffnung (10) ausreichende Größe für das Einführen des ersten Verbindungsstückes (1) hat, das eingeführte erste Verbindungsstück (1) dabei in Berührung mit den Flüssigkeit absorbierenden Materialien (8, 9) gebracht wird und die zweite Öffnung (11) eine ausreichende Größe für das Einführen des zweiten Verbindungsstückes (2) hat, dadurch gekennzeichnet, daß ein Bodenteil (7) lösbar an dem Seitenwandteil (6) befestigt ist, wobei die zweite Öffnung (11) frei ist, wenn das Bodenteil (7) von dem Seitenwandteil (6) gelöst ist, und wobei ein Teil (9) der absorbierenden Materialien (8, 9) an dem Bodenteil (7) befestigt ist, um das Endteil und das Innere des ersten Verbindungsstückes (1) zu desinfizieren.

2. Sterile Verbindungseinrichtung nach Anspruch 1, in der die Flüssigkeit absorbierenden Materialien (8, 9) eine Form haben, die mit jener des Verbindungsstückes (1) sich deckt.

3. Sterile Verbindungseinrichtung nach einem der Ansprüche 1 und 2, in der die Flüssigkeit absorbierenden Materialien (8, 9) ein an dem Seitenwandteil (6) befestigtes Teil (8) und ein an dem Bodenteil (7) befestigtes Teil (9) umfassen.

4. Sterile Verbindungseinrichtung nach einem der vorausgehenden Ansprüche, in der die Flüssigkeit absorbierenden Materialien (8, 9) Polyurethanschaumstoff umfassen.

5. Sterile Verbindungseinrichtung nach einem der vorausgehenden Ansprüche, in der die Form des Seitenwandteils (6) zylindrisch ist.

6. Sterile Verbindungseinrichtung nach einem der vorausgehenden Ansprüche, in der das Seitenwandteil (6) in das Bodenteil (7) eingepaßt ist.

**Revendications**

1. Dispositif de connexion stérile pour la connexion stérile d'un premier connecteur (1) à un second connecteur (2), comprenant un élément de paroi latérale (6), une première ouverture (10) dans l'élément de paroi latérale (6) en face d'une seconde ouverture (11), et des matériaux absorbants de liquide (8, 9) imprégnés d'une solution antiseptique, dans lequel la première ouverture (10) est d'une taille suffisant à l'insertion du premier connecteur (1), le premier connecteur (1) inséré étant alors mis en contact avec les matériaux absorbants de liquide (8, 9), et la seconde ouverture (11) étant d'une taille suffisant à l'insertion du second connecteur (2), caractérisé en ce qu'un élément de fond (7) est fixé de manière détachable à l'élément de paroi latérale (6) de sorte que la seconde ouverture (11) soit dégagée lorsque ledit élément de fond (7) est détaché de l'élément de paroi latérale (6) et de sorte qu'une partie (9) des matériaux absorbants (8, 9) soit fixée à l'élément de fond (7) pour désinfecter la partie d'extrémité et l'intérieur du premier connecteur (1).

2. Dispositif de connexion stérile selon la revendication 1, dans lequel les matériaux absorbants de liquide (8, 9) présentent une forme coïncidant avec celle du connecteur (1).

3. Dispositif de connexion stérile selon l'une quelconque des revendications 1 et 2 dans lequel les matériaux absorbants de liquide (8, 9) comprennent une partie (8) fixée à l'élément de paroi latérale (6) et une partie (9) fixée à l'élément de fond (7).

4. Dispositif de connexion stérile selon l'une quelconque des revendications précédentes, dans lequel les matériaux absorbants de liquide (8, 9) comprennent de la mousse de polyuréthanne.

5. Dispositif de connexion stérile selon l'une quelconque des revendications précédentes, dans lequel la forme de l'élément de paroi latérale (6) est cylindrique.

6. Dispositif de connexion stérile selon l'une quelconque des revendications précédentes, dans lequel l'élément de paroi latérale (6) s'emboîte dans l'élément de fond (7).

# FIG. 1

A

B

C

D

E

# FIG. 2

# FIG. 3